# NEW EUROPEAN PATENT SPECIFICATION

(11) **EP 0 199 475 B2**
(45) Date of publication and mention of the opposition decision: **17.04.1996**
(45) Mention of the grant of the patent: 30.08.1989
(21) Application number: 86302363.6
(22) Date of filing: 27.03.1986
(51) Int. Cl.: C07C 1/06, C01B 3/30

(54) **Fischer-Tropsch conversion of synthesis gas to hydrocarbons**
Verfahren zur Fischer-Tropsch-Umsetzung von Synthesegas in Kohlenwasserstoffen
Procédé de conversion Fischer-Tropsch de gaz de synthèse en hydrocarbures

(30) Priority: 02.04.1985 GB 8508613
(43) Date of publication of application: 29.10.1986
(73) Proprietor: The British Petroleum Company p.l.c., London EC2Y 9BU (GB)
(72) Inventor: Brophy, John Howard, Sunbury-on-Thames Middlesex, TW16 7LN (GB); Telford, Clive David, Sunbury-on-Thames Middlesex, TW16 7LN (GB); Font Freide, Josephus Johannes, Sunbury-on-Thames Middlesex, TW16 7LN (GB); Smith, David John Harry, Sunbury-on-Thames Middlesex, TW16 7LN (GB)
(74) Representative: Fawcett, Richard Fennelly

(56) References cited:
- EP-A- 0 070 690
- EP-A- 0 124 999
- EP-A- 0 163 385
- EP-A- 0 164 864
- EP-A- 0 178 853
- AU-A- 5 666 486
- DE-C- 896 792
- GB-A- 637 389
- GB-A- 652 477
- US-A- 2 253 607
- US-A- 2 327 066
- US-A- 2 414 276
- US-A- 2 437 600
- US-A- 2 473 995
- US-A- 2 497 761
- US-A- 2 539 434
- US-A- 2 563 607
- US-A- 2 606 939
- US-A- 2 738 360
- US-A- 4 547 525
- US-A- 4 678 860
- J. L. KUESTER et al, Diesel fuels from biomass derived synthesis gas, presented at the Catalytic Convention of Pyrolysis Gases Symposium, 1982, Aicheme Annnual Meeting, L.A., US
- CROWELL et al, Fischer Tropsch oil circulation processes, Ind. Eng. Chem., 42, no. 11, 2376, November 1950
- KRUIKOV et al, Transformations of butene under conditions of synthesis from CO and H2 over fused iron catalysts", IZV. AKAD. NAUK. SSSR, 58, 642, 1958
- Kuester statement dated 27. 7. 1990
- J. Amer. Chem. Soc., 52, 1930, SMITH et al, 3221-3232
- Adv. Chem.Ser., 178, 1979, DWYER et al, pp. 65-92
- Pan. Pac. Synfuels Conf., 1982, vol. 1, B11, NIIYAMA et al, pp. 197-203
- Des. Manag. Resource Recovery, 1980, vol. 1, chapter 15, KUESTER
- BALLIVET-TKATCHENKO et al, "Catalysis by zeolites", Elsevier, Ed Imelik et al, Amsterdam, 1980
- PICHLER et el, Erdöl U. Kohle-Erdgas Petrochemie, 23, 1970, 651-655
- PICHLER et al, Chem. Ing. Tech, 42, 18, pages 1162-1174, 1970
- KIBBY et al, "Hydrogenation of olefins in the presence of carbon monoxide on supported cobalt catalysts"; ACS Meeting, Philadelphia, USA, August 26-31, 1984
- DWYER et al, "The role of readsorption in determining the product distribution during CO hydrogenation over Fe single crystals", Journal of Catalysis, 56, 249-257, 1979

## Description

The present invention relates generally to a process for converting gaseous mixtures containing carbon monoxide and hydrogen into hydrocarbons by contact with Fischer-Tropsch catalysts. More particularly, the present invention relates to a process for the conversion of carbon monoxide, hydrogen and alkenes to hydrocarbons by contact with Fischer-Tropsch (FT) catalysts. Within the context of the present application the term « Fischer-Tropsch catalyst » includes related catalysts of the type generally referred to as « iso synthesis catalysts » and derivatives thereof.

The Fischer-Tropsch (F-T) synthesis originated in Germany in the 1920's, reached its zenith in Germany during World War II as a supplier principally of motor fuels and now survives only in South Africa in combination with a coal gasification process at the Sasol works. Immediately following World War II, most industrial nations were involved in F-T research but interest began to decline with the advent of cheap oil and gas. In recent years however interest in the F-T process has revived and considerable research has been directed towards the development of new and improved catalysts. As a consequence, the range of catalytically active F-T metal components has been extended beyond iron and cobalt, the basis of the original catalysts.

A demand exists for a Fischer-Tropsch process for converting synthesis gas to produce high yields of liquid hydrocarbon products, at the same time minimising the production of C₁ products and maximising the total carbon conversion and productivity and in particular the conversion and productivity to C₃ or greater hydrocarbons.

We have found that this demand can be to some extent met by the provision in the synthesis gas feed to a Fischer-Tropsch process of a defined proportion of an alkene and the use of specific reaction conditions, particularly if the Fischer-Tropsch catalyst incorporates a solid acidic component.

Processes in which mixtures of synthesis gas and unsaturated hydrocarbons are reacted over a catalyst are known in the art.

Thus, for example, USP-A-2 606 939 describes the conversion of olefinic feedstock containing carbon monoxide and hydrogen, the olefin being present in an amount greater than 60 per cent, preferably greater than 70 % by volume, by polymerisation/hydropolymerisation over a promoted iron catalyst to higher olefinic hydrocarbons. This represents a disclosure of an olefin conversion process, as opposed to a synthesis gas conversion process.

DE-C-896 792 discloses a method for the production of olefin-rich hydrocarbon mixtures by the catalytic reaction of gaseous mixtures of carbon monoxide and hydrogen that also contain unsaturated hydrocarbons, using cobalt catalysts at normal atmospheric pressure at a temperature of 185 to 225°C according to Patent 764 165 characterised by the fact that, in addition to acetylene, hydrocarbons with a double bond, particularly ethylene, are also added to the synthesis gas.

The present invention provides a process for the production of a hydrocarbon product by contacting a gaseous mixture comprising carbon monoxide, hydrogen and ethene with a Fischer-Tropsch catalyst under Fischer-Tropsh conditions characterised in that the Fischer-Tropsch catalyst comprises one or more metals of Group VIII of the Periodic Table of the Elements or molybdenum, tungsten or rhenium, in the form of the elemental metal, an oxide or a sulphide and incorporates a solid acidic component, or comprises an oxide of either zinc, gallium or indium together with a porous crystalline tectometallosilicate, the ethene is present in an amount of from 2 to 15% by volume and the Fischer-Tropsch conditions are a temperature in the range from 175 to 450°C and a pressure in the range from 20 to 100 bar

Suitable F-T catalyst metals include for example iron, cobalt, nickel, molybdenum, tungsten, rhenium, ruthenium, palladium, rhodium, osmium, iridium and platinum, preferably iron, cobalt or ruthenium. The metal is in the form of the elemental metal, an oxide or a sulphide. The catalyst may suitably include a promoter which may be an alkali metal, an alkaline earth metal or a rare earth metal. Examples of suitable promoters include the hydroxide, oxide or salt of lithium, sodium, potassium, rubidium, cesium, magnesium, calcium, strontium, barium and thorium. The support may be, for example, alumina, carbon, silica, zirconia, titania, magnesia, ceria or mixtures thereof. Iso-synthesis catalysts generally comprise non-reducible oxides, for example thoria, zirconia, alumina, ceria, and gallia. Conventional techniques, such as for example, impregnation, may be used for the preparation of such catalysts.

The F-T catalyst incorporates, suitably either by way of a support or as an intimate admixture therewith, a solid acidic component. Suitable acidic components include for example, silica/alumina, layered clays, pillared layered clays, and crystalline tectometallosilicate, for example gallosilicate and aluminosilicate zeolites. Preferred are crystalline gallosilicate and aluminosilicate zeolites having a high silica content (silica to alumina molar ratio greater than 12:1), suitably in the hydrogen form. An example of a suitable zeolite is an MFI-type zeolite (as described in the book by Meier, W.M. and Olson, D.H. entitled Atlas of Zeolite Structure Types, published by the Structure Commission of the International Zeolite Association, 1978, and distributed by Polycrystal Book Service, Pittsburgh, Pa, USA), for example ZSM-5 as described and claimed in US Patent No. 3 702 886. The hydrogen form of a crystalline aluminosilicate zeolite may suitably be prepared by techniques well-known in the zeolite art.

Examples of catalysts suitable for use in the process of the present invention include (i) an oxide of zinc, gallium or indium together with a porous crystalline tectometallosilicate as described in our European patent application publication No. EP-A-0 124 999, for example a thoria/gallia/MFI-type crystalline aluminosilicate zeolite, (ii) a composition comprising ruthenium/ceria as described in our published European application No. 169 743, preferably in combination with an MFI-type crystalline aluminosilicate zeolite and (iii) an iron/crystalline acidic aluminosilicate zeolite catalyst as described, for example, in USP-A-4 298 695.

In one embodiment of the present invention, the mixture of carbon monoxide and hydrogen and the alkene are derived from separate sources and are subsequently combined in the presence of the F-T catalyst. Mixtures of gases principally comprising carbon monoxide and hydrogen, possibly containing also carbon dioxide, nitrogen and methane for example are generally referred to in the art as synthesis gas. Synthesis gas may in theory be obtained from any carbonaceous source, of which coal, natural gas and high molecular weight hydrocarbon residues may be mentioned as examples. Thus, the synthesis gas may suitably be obtained by the gasification of coal using technology well-known in the art, for example the gasifier process developed by Lurgi Kohle und Mineraloeltechnik GmbH. Alternatively, the synthesis gas may be obtained by the steam reforming of hydrocarbon feeds, for example sulphur-free natural gas or paraffinic naphtha, in the presence or absence of a reforming catalyst. In another alternative, synthesis gas may be produced by partial oxidation of a hydrocarbon feed, wherein the hydrocarbon feed is introduced into an oxidation zone maintained in a fuel rich mode, in the absence of a catalyst and the presence or absence of steam. Catalytic autothermal reforming of hydrocarbon liquids, i. e. partial oxidation in the presence of added steam, may also be used to produce the synthesis gas necessary for the performance of the invention.

An example of a suitable method of preparing synthesis gas is described in our copending European application publication No. 164 864 which provides a process for the production of synthesis gas in which (a) a saturated hydrocarbon and an oxygen containing gas having a ratio of hydrocarbon to oxygen of greater than the stoichiometric ratio for complete combustion are introduced into a bed of particulate material, the bed comprising material which is catalytically active for partial oxidation and/or steam reforming reactions, (b) the upward flow rate of the hydrocarbon/oxygen containing gas stream being sufficiently large to cause a spouting action of the bed material, (c) the hydrocarbon and oxygen reacting together and (d) the products of the reaction being withdrawn.

The alkene may be substantially pure or may be present in a mixture with other materials, for example hydrogen and paraffinic hydrocarbons, in a petroleum refinery fraction or other fraction. A suitable fraction rich in alkenes is for example the product derived from thermal cracking of C₂ to C₄ alkanes or other petroleum fractions such as naphtha.

The alkene may be combined with the synthesis gas feed to a reactor containing an F-T catalyst or may be fed separately to such a reactor.

According to a preferred embodiment of the process of the present invention, in a first step there is formed a gaseous mixture comprising carbon monoxide, hydrogen and an alkene and in a second step the mixture formed in the first step is converted to a product comprising hydrocarbons by contact with a Fischer-Tropsch catalyst under Fischer-Tropsch conditions.

An advantage of operating the process in this manner is that the total carbon converted per pass can be greatly increased as compared with making the carbon monoxide/hydrogen separately and converting that over an F-T catalyst. Furthermore, certain catalysts show a synergistic effect i. e. the CO conversion is increased in the presence of the alkene and/or the proportion of CO₂ and methane produced are reduced.

The first step wherein there is formed a gaseous mixture comprising carbon monoxide, hydrogen and an alkene may be performed in a variety of ways. It may, for example, be formed by the partial oxidation/cracking of one or more gaseous hydrocarbons or readily vapourisable hydrocarbons. One suitable method involves the partial oxidation of gaseous or vapourised hydrocarbons with oxygen by preheating the reactants together or separately, supplying the mixture to a reaction chamber, reacting them in a flame and rapidly cooling the reaction gases, as described in British Patent No. 835676 for example.

A particularly preferred method is described in our pending published European application No. 163385 which provides a process for the production of synthesis gas and higher hydrocarbons in which (a) a saturated hydrocarbon and an oxygen containing gas having a ratio of hydrocarbon to oxygen of greater than the stoichiometric ratio for complete combustion are introduced into a bed of an inert particulate material, (b) the upward flow rate of the hydrocarbon/oxygen containing gas stream being sufficiently large to fluidise or to produce a spouting action of the bed material whereby at least a part of the particulate material is thrown up above the bed surface and subsequently falls back into the bed, (c) the hydrocarbon and oxygen containing gas being ignited and reacted together and (d) the products of the reaction being withdrawn.

Yet another method is described in our copending published European application publication No. 178853 which provides a process for the production of synthesis gas and/or higher hydrocarbon in which (a) a saturated hydrocarbon and an oxygen containing gas having a ratio of hydrocarbon to oxygen of greater than the stoichiometric ratio for complete combustion are introduced together with hydrogen into a bed of particulate material (b) the upward flow rate of the gases being sufficiently large to cause a spouting action of the bed material above the slumped level of the bed (c) the hydrocarbon, oxygen and hydrogen reading together and (d) the products of the reaction being withdrawn.

Other methods of performing the first step to provide a feedstock comprising carbon monoxide, hydrogen and an alkene will be readily apparent to those skilled in the art.

It is preferred that the gaseous mixture produced in the first step of the process be transferred without substantial intermediate treatment directly to the second step. However, in certain circumstances it may be desirable to subject the gaseous mixture formed in the first step to, for example, an intermediate step in which water is removed from the gaseous mixture and/or an intermediate step in which alkynes present in the gaseous mixture are selectively hydrogenated to alkenes.

The Fischer-Tropsch reaction conditions are a temperature in the range 175 to 450 °C and a pressure in the range from 20 to 100 bars. The optimum reaction conditions will vary from catalyst to catalyst but will be within the aforesaid ranges.

The process is preferably operated on a continuous basis.

The nature of the hydrocarbon product will depend amongst other factors on the particular F-T catalyst selected, the specific reaction conditions employed and upon the particular composition of the gaseous mixture fed to the F-T catalyst.

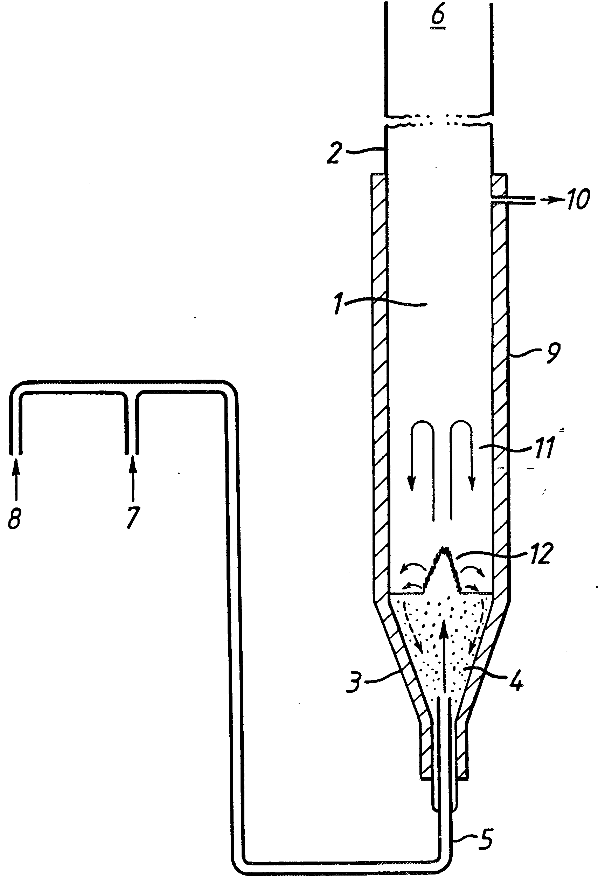

The invention will now be further illustrated by way of example only and with reference to the accompanying drawing.

### (A) Production of CO, H₂, Alkene mixture

The drawing shows a schematic layout of a reactor and ancillary equipment.

The reactor 1 takes the form of a lagged elongate quartz column 2 having a conical base portion 3, the angle of the cone from the vertical being 20°. The base portion of the reactor contains a slumped bed 4 of particulate material having a particle size of the order 1-1.5 mm diameter. The particulate materials were crushed firebrick, silicon carbide, quartz and zirconia. The base of the column is adapted to receive a nozzle 5 for the introduction of reactants. The nozzle outlet may be adjusted vertically within the bed of particulate material. The upper portion 6 of the reactor isopen to form an outlet for withdrawal of the product gases. A fine 10 enables samples of the products to be withdrawn from the product gas stream.

The nozzle 5 is connectable to a supply of air 7 or other oxygen containing gas under appropriate pressure and to a supply of methane 8. A suitable supply may comprise cylinders of hydrocarbon e. g. methane, and air or oxygen linked to the nozzle through a mixer and gas pressure and flow rate measuring devices such as manometers and rotameters (not shown).

The reactor may also have an additional nozzle or nozzles for supplying further methane or other hydrocarbon to the bed (not shown in the drawing). The reactor 1 is lagged with a suitable insulating material 9.

A number of techniques may be used for start up of the reactor. In the present example, the ignition source was a gas burner (not shown) located at the outlet portion 6 of the column.

During start up of the reactor, a pre-mixed gas stream of hydrocarbon and air was passed under pressure to the nozzle 5 in the base portion 3 of the column. The velocity of the gas stream was sufficient to cause a fountain 11 of bed particles in the freeboard above the bed.

The gas stream used was very fuel rich and consequently the gas mixture was ignited by the gas burner and a flame stabilised at the exit of the reactor. The air flowrate was increased, bringing the mixture closer to stoichiometric, until the flame began to move slowly back down the reactor. A flame was stabilised at the surface of the slumped bed and the fuel flowrate reduced slightly to obtain a near stoichiometric mixture. When the bed temperatures had equilibrated, the fuel flowrate was increased and a low flowrate of oxygen was added to the bed. The air flowrate was then reduced and both the fuel and the oxygen increased to maintain the stable flame and the spouting action of the bed. This procedure was repeated until the feed composition was entirely fuel and oxygen The total feed mixture was always maintained on the rich side of stoichiometric close to or beyond the rich limit of flammability. At atmospheric pressure, the rich limit of flammability corresponds to a methane/oxygen mole ratio of 1.5.

The constituents and composition of the reactant gases were ascertained by means of conventional techniques. This procedure was repeated for a number of fuel rich hydrocarbon/oxygen reactant compositions and different particulate bed materials. The products obtained from the reaction may include carbon monoxide and hydrogen (synthesis gas), acetylene, and ethylene.

Table 1 shows results for the reaction of methane and oxygen in a reactor using various bed materials. The bed materials generally had particle size of 1 to 1.5 mm diameter and were crushed firebrick, quartz and silicon carbide. The increased carbon molar selectivities to C₂ and higher hydrocarbons is shown for all the bed materials for fuel rich conditions as the feed composition is made increasingly fuel rich up to and beyond the rich limit of flammability.

Table 2 shows results for the reaction of methane and air in a reactor. The bed material used was zirconia spheres of the order 1-1.2 mms diameter. The carbon molar selectivities and feed conversions achieved are similar to those obtained with oxygen.

Calculations have shown that the residence time of the reactant gases in the hot zone is desirably less than 1 millisecond in order to avoid an undesirably high degree of cracking of the products to soot or other compounds. It may be possible to use longer residence times at low or atmospheric pressures.

Aternatively a lower velocity of the gas stream may be used so as to cause fluidisation of the bed material and/or surface bubbling of the bed material. The fluidisation and/or bubbling is sufficient to cause the particulate material to be thrown up into the freeboard, the material retuming to the bed. The bubbling may also be associated with total fluidisation of the bed.

Table 3 shows results obtained in a fluidised bed reactor with a methane/oxygen feed. The bed comprised 0.25-0.85 mm particles of zirconia. The reactant gases were fed to the reactor through a distributor plate at a sufficient velocity to attain turbulent fluidisation with bed material thrown randomly into the gas phase or freeboard above the bed.

Experiments with fuel rich mixtures close to or beyond the limit of flammability using conventional fluidised bed operation at one bar pressure and with an inert bed material generally resulted in flame lift off or instability. At the onset of surface bubbling (or localised spouting) a more stable combustion regime ensued and partial oxidation with production of CO/H₂ and C₂'s resulted. This effect persisted into the complete spouting regime. It is believed that the stable combustion of mixtures close to or beyond the limit of flammability results from the countercurrent heat transfer between the descending hot particulate material and the ascending gases thereby enabling pre-heat of incoming feed gases.

As the pressure of operation is increased the upper limit of flammability widens. Thus, the need for particulate heat recirculation for stable combustion is greater at lower operating pressures than at higher operating pressures. However by application of the present invention the limit of flammability can be further extended at increased pressures by particulate heat recirculation in a similar way as at atmospheric pressure.

### (See Tables 1-3 pages 7 and 8)

### (B) Conversion of CO, H₂ and alkene mixture

### Comparison Test 1

A tubular reactor was loaded with 10mls of a ThO₂/Ga₂O₃/H-MFI zeolite catalyst (bound with silica) which had previously been run for 20 hours with a syngas feed at about 400 °C. Feed gas (syngas comprising 65 % volume hydrogen and 35 % carbon monoxide designated feed gas X) was fed to the reactor at a gas hourly space velocity (GHSV) of 2,000 h⁻¹. The reactor was heated externally to maintain an average bed temperature of 400 °C at a pressure of 50 barg. The reaction products were analysed by gas chromatography. The conditions and results are summarised in Table 4.

This is not an example according to the invention because an unsaturated hydrocarbon was not present.

### Example 1

Following comparison Test 1, a feed stream Y containing hydrogen 57 % volume, carbon monoxide 33,5 % and ethylene 9,5 % (i. e. simulating in composition the product as produced in A) was fed to the reactor under essentially identical conditions. The conditions and results are summarised in Table 4.

The presence of ethylene in the feed clearly has a synergistic effect:- ethylene is converted completely while carbon monoxide conversion is increased and overall carbon conversion is increased. Methane and carbon dioxide selectivities are reduced. The net result is a five fold increase in productivity of C₃ and higher hydrocarbons.

### Comparison Test 2

A reactor was charged with 10mls of a Ru/K/CeO₂ catalyst. Feed gas X (cf Comp Test 1) was passed over the catalyst at 40 bar, 2 500 GHSV, while the maximum bed temperature was maintained at 331 °C. Results are given in Table 4.

This is not an example according to the invention because of the absence of an unsaturated hydrocarbon.

### Example 2 (Comparative)

Ethylene containing syngas (Feed gas Y of Example 1) was substituted for the syngas feed (X) over the catalyst of Comparison Test 2. The results are shown in Table 4. Under identical conditions there is a significant increase in C₃+ productivity due to the addition of ethylene, and a reduction in methane and carbon dioxide selectivities. However ethylene conversion is relatively low over this catalyst.

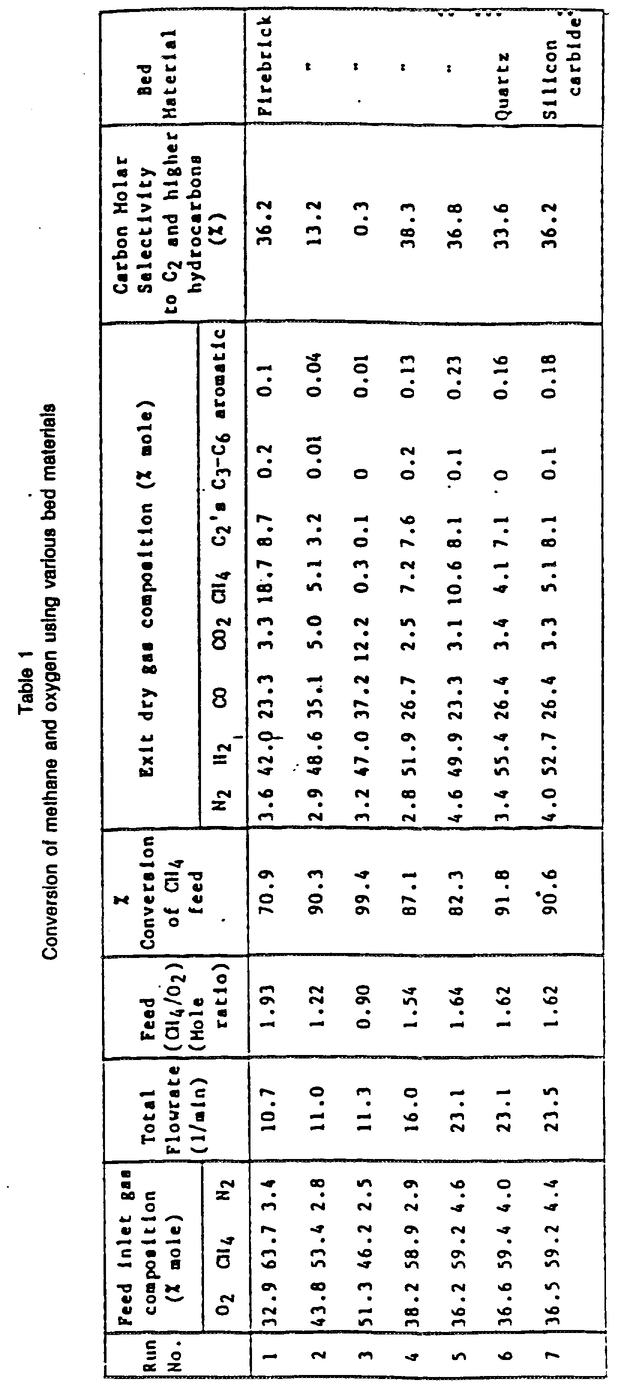

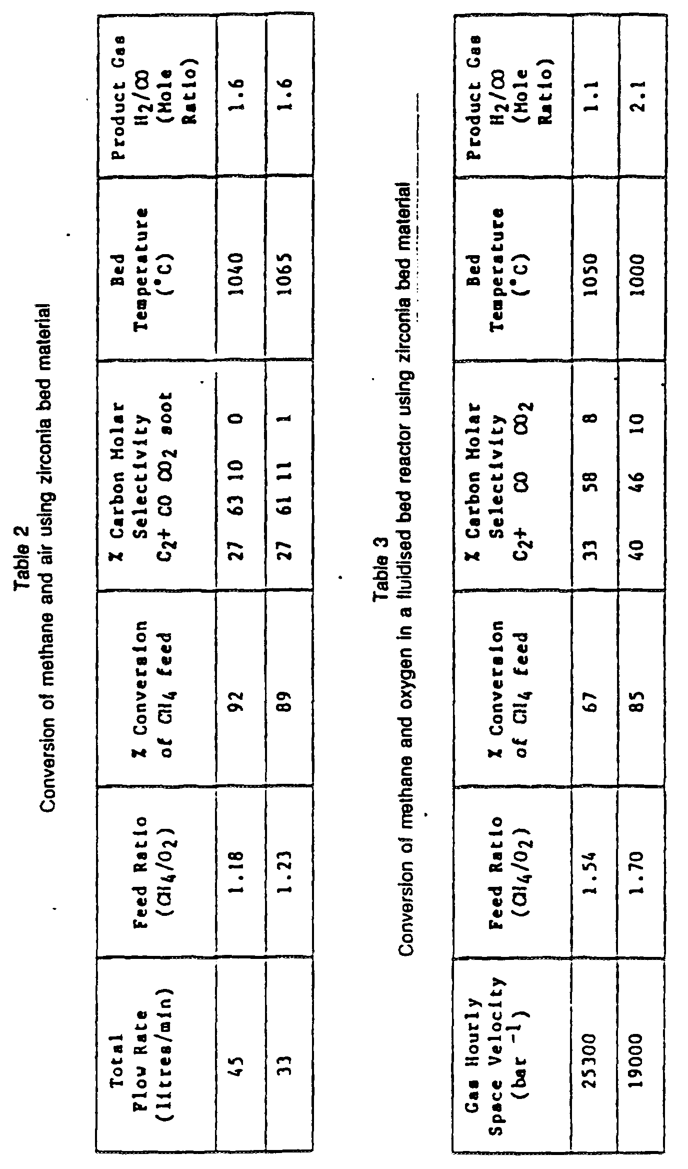

### Comparison Test 3

The reactor was charged with a physical mixture of Ru/K/CeO₂ (6mls) and silica bound zeolite (H-MFI) (4mls). Feed gas X (cf Comp. Test 1) was fed to the reactor at 40 bar, 2500 GHSV, 370 °C. Results are shown in Table 4.

This is not an example according to the invention because of the absence of unsaturated hydrocarbon.

### Example 3

Feed gas Y (cf Example 1) was substituted for Feed gas X in Comparison Test 3 and the reaction products over the catalyst used in Comparison Test 3 were analysed. Results in Table 4 show that there is a greater improvement in C₃+ productivity in this example where an acidic zeolite has been incorporated into the catalyst bed and a greater increase in total carbon conversion. Ethylene conversion is considerably higher while at the same time, selectivity to C₃+ hydrocarbons is improved.

### Comparison Test 4

The procedure of Comparison Test 1 was repeated except that instead of the ThO₂/GaO₂O₃/H-MFI zeolite catalyst there was used a magnetite/H-MFI catalyst. The reaction conditions and the results obtained are given in Table 4.

This is not an example according to the invention because of the absence of an unsaturated hydrocarbon in the feed.

### Example 4

Comparison Test 4 was repeated except that instead of feed gas X there was used feed gas Y.

The results are given in Table 4.

From the above results it is clear that olefin conversion is maximised in the presence of an acidic (zeolite) component.

### (See Table 4 page 10.)

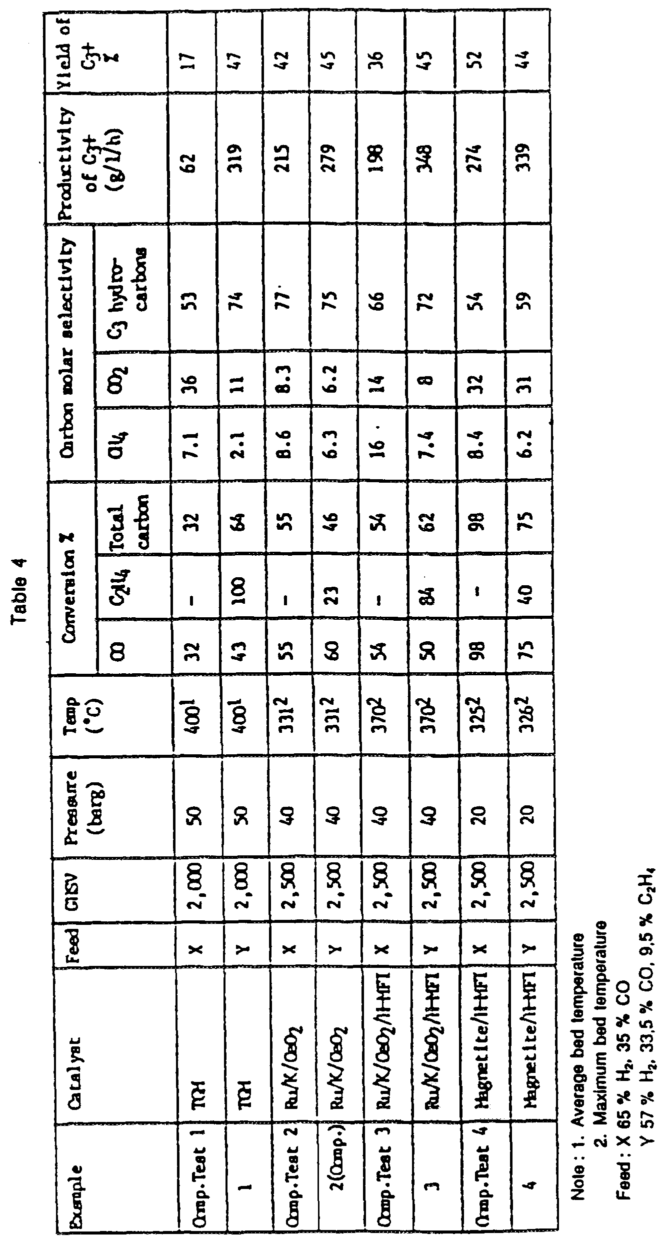

## Claims

1. A process for the production of a hydrocarbon product by contacting a gaseous mixture comprising carbon monoxide, hydrogen and ethene with a Fischer-Tropsch catalyst under Fischer-Tropsch conditions characterised in that the Fischer-Tropsch catalyst comprises one or more metals of Group VIII of the Periodic Table of the Elements or molybdenum, tungsten or rhenium, in the form of the elemental metal, an oxide or a sulphide and incorporates a solid acidic component, or comprises an oxide of either zinc, gallium or indium together with a porous crystalline tectometallosilicate, the ethene is present in an amount of from 2 to 15% by volume and the Fischer-Tropsch conditions are a temperature in the range from 175 to 450°C and a pressure in the range from 20 to 100 bar.

2. A process according to claim 1 wherein the Group VIII metal is either iron, cobalt or ruthenium.

3. A process according to either claim 1 or claim 2 wherein the Group VIII metal or metals is supported on either alumina, carbon, silica, zirconia, titania, magnesia, ceria or gallia.

4. A process according to any one of the preceding claims wherein the solid acidic component is a crystalline aluminosilicate or gallosilicate zeolite having a silica to alumina molar ratio greater than 12:1.

5. A process according to claim 4 wherein the zeolite is in the hydrogen form.

6. A process according to claim 1 wherein the Fischer-Tropsch catalyst is a composition comprising ruthenium and ceria.

7. A process according to claim 6 wherein the ruthenium and ceria are combined with an MFI-type crystalline aluminosilicate zeolite.

8. A process according to claim 1 wherein the Fischer-Tropsch catalyst is an iron/ crystalline acidic aluminosilicate zeolite.

9. A process according to any one of the preceding claims wherein the synthesis gas is obtained by a process in which (a) a saturated hydrocarbon and an oxygen-containing gas having a ratio of hydrocarbon to oxygen of greater than the stoichiometric ratio for complete combustion are introduced into a bed of particulate material, the bed comprising material which is catalytically active for partial oxidation and/or steam reforming reactions, (b) the upward flow rate of the hydrocarbon/oxygen containing gas stream being sufficiently large to cause a spouting action of the bed material, (c) the hydrocarbon and oxygen reacting together, and (d) the products of the reaction being withdrawn.

10. A process according to any one of claims 1 to 8 wherein a gaseous mixture comprising carbon monoxide, hydrogen and ethene is formed in a first step and the mixture formed in the first step is converted to a product comprising hydrocarbons in a second step.

11. A process according to claim 10 wherein the gaseous mixture is obtained by partial oxidation/cracking of one or more gaseous hydrocarbons or readily vapourisable hydrocarbons.

12. A process according to claim 11 wherein the gaseous mixture is obtained by a process in which (a) a saturated hydrocarbon and an oxygen containing gas having a ratio of hydrocarbon to oxygen of greater than the stoichiometric ratio for complete combustion are introduced into a bed of an inert particulate material, (b) the upward flow rate of the hydrocarbon/oxygen containing gas stream being sufficiently large to fluidise or to produce a spouting action of the bed material whereby at least a part of the particulate material is thrown up above the bed surface and subsequently falls back into the bed, (c) the hydrocarbon and oxygen containing gas being ignited and reacted together, and (d) the products of the reaction being withdrawn.

13. A process according to claim 11 wherein the gaseous mixture is obtained by a process in which (a) a saturated hydrocarbon and an oxygen containing gas having a ratio of hydrocarbon to oxygen of greater than the stoichiometric ratio for complete combustion are introduced together with hydrogen into a bed of particulate material, (b) the upward flow rate of the gases being sufficiently large to cause a spouting action of the bed material above the slumped level of the bed, (c) the hydrocarbon, oxygen and hydrogen reacting together, and (d) the products of the reaction being withdrawn.

## Patentansprüche

1. Verfahren zur Herstellung eines Kohlenwasserstoffproduktes durch In-Kontakt-Bringen einer gasförmigen Mischung umfassend Kohlenmonoxid, Wasserstoff und Ethen mit einem Fischer-Tropsch-Katalysator unter Fischer-Tropsch-Bedingungen, dadurch gekennzeichnet, daß der Fischer-Tropsch-Katalysator ein oder mehrere Metalle der Gruppe VIII des Periodensystems der Elemente oder Molybdän, Wolfram oder Rhenium in der Form des elementaren Metalls, eines Oxids oder eines Sulfids umfaßt und eine feste, saure Komponente enthält oder ein Oxid von entweder Zink, Gallium oder Indium zusammen mit einem porösen kristallinen Tectometallosilicat umfaßt, das Ethen in einer Menge von 2 bis 15 Volumenprozent vorliegt und die Fischer-Tropsch-Bedingungen eine Temperatur in dem Bereich von 175 bis 450°C und ein Druck in dem Bereich von 20 bis 100 bar sind.

2. Verfahren nach Anspruch 1, worin das Metall der Gruppe VIII entweder Eisen, Kobalt oder Ruthenium ist.

3. Verfahren nach Anspruch 1 oder 2, worin das Metall oder die Metalle der Gruppe VIII getragen werden auf entweder Aluminiumoxid, Kohlenstoff, Siliciumdioxid, Zirkonoxid, Titanoxid, Magnesiumoxid, Ceroxid oder Galliumoxid.

4. Verfahren nach einem der vorhergehenden Ansprüche, worin die feste, saure Komponente ein kristalliner Aluminosilicat- oder Gallosilicat-Zeolith mit einem Siliciumdioxid-zu-Aluminiumoxid-Molverhältnis größer als 12:1 ist.

5. Verfahren nach Anspruch 4, worin der Zeolith in der Wasserstofform ist.

6. Verfahren nach Anspruch 1, worin der Fischer-Tropsch-Katalysator eine Zusammensetzung umfassend Ruthenium und Ceroxid ist.

7. Verfahren nach Anspruch 6, worin das Ruthenium und Ceroxid mit einem kristallinen Aluminosilicat-Zeolith vom MFI-Typ kombiniert sind.

8. Verfahren nach Anspruch 1, worin der Fischer-Tropsch-Katalysator ein Eisen/kristallines, saures Aluminosilicat-Zeolith ist.

9. Verfahren nach einem der vorhergehenden Ansprüche, worin das Synthesegas erhalten wird durch ein Verfahren, bei dem (a) ein gesättigter Kohlenwasserstoff und ein sauerstoffhaltiges Gas mit einem Verhältnis von Kohlenwasserstoff zu Sauerstoff größer als das stöchiometrische Verhältnis für vollständige Verbrennung in ein Bett von teilchenförmigem Material eingeführt werden, wobei das Bett Material umfaßt, das katalytisch aktiv für teilweise Oxidation und/oder Dampf-Umformungsreaktionen ist, (b) der Aufwärtsdurchsatz des Kohlenwasserstoff/Sauerstoff enthaltenden Gasstromes ausreichend groß ist, um eine Herausspritzwirkung des Bettmaterials zu bewirken, (c) der Kohlenwasserstoff und Sauerstoff miteinander reagieren und (d) die Reaktionsprodukte entnommen werden.

10. Verfahren nach einem der Ansprüche 1 bis 8, worin eine gasförmige Mischung umfassend Kohlenmonoxid, Wasserstoff und Ethen in einem ersten Schritt gebildet wird und die in dem ersten Schritt gebildete Mischung zu einem Produkt umfassend Kohlenwasserstoffe in einem zweiten Schritt umgewandelt wird.

11. verfahren nach Anspruch 10, worin die gasförmige Mischung durch teilweise Oxidation/Cracken von einem oder mehreren gasförmigen Kohlenwasserstoffen oder leicht verdampfbaren Kohlenwasserstoffen erhalten wird.

12. Verfahren nach Anspruch 11, worin die gasförmige Mischung durch ein Verfahren erhalten wird, in dem (a) ein gesättigter Kohlenwasserstoff und ein Sauerstoff enthaltendes Gas mit einem Verhältnis von Kohlenwasserstoff zu Sauerstoff von größer als dem stöchiometrischen Verhältnis zur vollständigen Verbrennung in ein Bett eines inerten teilchenförmigen Materials eingeführt wird, (b) der Aufwärtsdurchsatz des Kohlenwasserstoff/Sauerstoff enthaltenden Gasstromes ausreichend groß ist, um zu fluidisieren oder eine Herausspritzwirkung des Bettmaterials zu produzieren, wobei mindestens ein Teil des teilchenförmigen Materials über die Bettoberfläche hochgeworfen wird und anschließend in das Bett zurückfällt, (c) das Kohlenwasserstoff und Sauerstoff enthaltende Gas entzündet und miteinander umgesetzt wird und (d) die Reaktionsprodukte entnommen werden.

13. Verfahren nach Anspruch 11, worin die gasförmige Mischung durch ein Verfahren erhalten wird, in dem (a) ein gesättigter Kohlenwasserstoff und ein Sauerstoff enthaltendes Gas mit einem Verhältnis von Kohlenwasserstoff zu Sauerstoff größer als das stöchiometrische Verhältnis für vollständige Verbrennung zusammen mit Wasserstoff in ein Bett von teilchenförmigem Material eingeführt werden, (b) der Aufwärtsdurchsatz des Gases ausreichend groß ist, um eine Herausspritzwirkung des Bettmaterials über das abgesetzte Niveau des Bettes zu bewirken, (c) der Kohlenwasserstoff, Sauerstoff und Wasserstoff miteinander reagieren und (d) die Produkte der Reaktion entnommen werden.

## Revendications

1. Procédé de production d'un produit hydrocarboné, par mise en contact d'un mélange gazeux comprenant du monoxyde de carbone, de l'hydrogène et de l'éthène (éthylène) avec un catalyseur de Fischer-Tropsch dans les conditions de Fischer-Tropsch, procédé caractérisé en ce que le catalyseur de Fischer-Tropsch comprend un ou plusieurs métaux du groupe VIII du Tableau Périodique des Eléments ou du molybdène, du tungstène ou du rhénium, sous forme de métal élémentaire, d'un oxyde ou d'un sulfure, et le catalyseur incorpore un constituant acide solide ou comprend un oxyde de zinc, de gallium ou d'indium avec un tectométallosilicate cristallin poreux ; l'éthène est présent en une quantité de 2 à 15 % en volume et les conditions de Fischer-Tropsch consistent en une température comprise entre 175 et 450 °C et en une pression comprise entre 20 et 100 bars.

2. Procédé selon la revendication 1, dans lequel le métal du groupe VIII est du fer, du cobalt ou du ruthénium.

3. Procédé selon la revendication 1 ou la revendication 2, dans lequel le métal ou les métaux du groupe VIII est ou sont supportés sur de l'alumine, sur du carbone, sur de silice, sur de l'oxyde de zirconium, sur de l'oxyde de titane, sur de la magnésie, sur de l'oxyde de cérium ou de l'oxyde de gallium.

4. Procédé selon l'une quelconque des revendications précédentes, dans lequel le constituant acide solide est un aluminosilicate cristallin ou un gallosilicate zéolitique ayant un rapport molaire de la silice à l'alumine supérieur à 12:1.

5. Procédé selon la revendication 4, dans lequel la zéolite est sous forme hydrogénée.

6. Procédé selon la revendication 1, dans lequel le catalyseur de Fischer-Tropsch est une composition comprenant du ruthénium et de l'oxyde de cérium.

7. Procédé selon la revendication 6, dans lequel le ruthénium et l'oxyde de cérium sont combinés avec un aluminosilicate zéolitique cristallin de type MFI.

8. Procédé selon la revendication 1, dans lequel le catalyseur de Fischer-Tropsch est une zéolite fer/aluminosilicate acide cristallin.

9. Procédé selon l'une quelconque des revendications précédentes, dans lequel le gaz de synthèse est obtenu par un procédé dans lequel (a) un hydrocarbure saturé et un gaz contenant de l'oxygène, présentant un rapport de l'hydrocarbure à l'oxygène supérieur au rapport stoechiométrique pour une combustion complète, sont introduits dans un lit d'une matière particulaire, le lit comprenant un matériau qui est catalytiquement actif pour des réactions d'oxydation partielle et/ou de reformage à la vapeur d'eau, (b) le débit ascendant du courant gazeux contenant de l'hydrocarbure/oxygène étant suffisamment grand pour provoquer une action de jaillissement du matériau du lit, (c) l'hydrocarbure et l'oxygène réagissant ensemble, et (d) les produits de la réaction étant soutirés.

10. Procédé selon l'une quelconque des revendications 1 à 8, dans lequel un mélange gazeux comprenant du monoxyde de carbone, de l'hydrogène et de l'éthène est formé dans une première étape, et le mélange formé dans la première étape est converti, dans une seconde étape, en un produit comprenant des hydrocarbures.

11. Procédé selon la revendication 10, dans lequel le mélange gazeux est obtenu par oxydation partielle/craquage d'un ou plusieurs hydrocarbures gazeux ou d'hydrocarbures facilement vaporisables.

12. Procédé selon la revendication 11, dans lequel le mélange gazeux est obtenu par un procédé dans lequel (a) un hydrocarbure saturé et un gaz contenant de l'oxygène et ayant un rapport entre l'hydrocarbure et l'oxygène supérieur au rapport stoechiométrique pour une combustion complète, sont introduits dans un lit d'une matière particulaire inerte, (b) le débit d'écoulement ascendant du courant hydrocarbure/gaz contenant de l'oxygène étant suffisamment grand pour fluidiser ou pour produire une action de jaillissement du matériau du lit, de sorte qu'une partie au moins de la matière particulaire est soulevée au-dessus de la surface du lit et retombe ensuite dans le lit, (c) l'hydrocarbure et le gaz contenant de l'oxygène étant allumés et réagissant ensemble, et (d) les produits de la réaction étant soutirés.

13. Procédé selon la revendication 11, dans lequel le mélange gazeux est obtenu par un procédé dans lequel (a) un hydrocarbure saturé et un gaz contenant de l'oxygène, présentant un rapport de l'hydrocarbure à l'oxygène supérieur au rapport stoechiométrique pour une combustion complète, sont introduits ensemble avec l'hydrogène dans un lit de matière particulaire, (b) le débit d'écoulement ascendant des gaz étant suffisamment grand pour provoquer une action de jaillissement du matériau du lit au-delà du niveau incliné du lit, (c) l'hydrocarbure, l'oxygène et l'hydrogène réagissant ensemble, et (d) les produits de la réaction étant soutirés.
